Europäisches Patentamt

**European Patent Office**

**Office européen des brevets**

(19)

(11) Publication number: **0 131 418**
**A1**

(12) # EUROPEAN PATENT APPLICATION

(21) Application number: **84304469.4**

(22) Date of filing: **29.06.84**

(51) Int. Cl.⁴: **C 12 N 15/00**
C 12 P 21/02, C 12 N 9/20
A 61 K 37/54, C 12 N 1/16

(30) Priority: **01.07.83 GB 8317989**
**05.09.83 GB 8323759**

(43) Date of publication of application:
**16.01.85 Bulletin 85/3**

(84) Designated Contracting States:
**AT BE CH DE FR GB IT LI LU NL SE**

(71) Applicant: **CELLTECH LIMITED**
**244-250 Bath Road**
**Slough Berkshire SL1 4DY(GB)**

(72) Inventor: **Williamson, Robert**
**71A Flask Walk Highgate**
**London NW3, 1ET(GB)**

(72) Inventor: **Carey, Norman Henry**
**Russells Close High Street**
**Chinnor Oxfordshire, OX9 4DJ(GB)**

(74) Representative: **Votier, Sidney David et al,**
**CARPMAELS & RANSFORD 43, Bloomsbury Square**
**London WC1A 2RA(GB)**

(54) **Proteins, pharmaceutical compositons, genes, vectors, host organisms and processes for their production.**

(57) A rat or human lingual lipase protein for use in the treatment of lipase deficiency. A process is described for producing lingual lipase using recombinant DNA technoloy to produce a host organism (for example *E.coli*) capable of producing a precursor of the lingual lipase which may be cleaved to yield the lingual lipase. The host organism is transformed with a vector including a gene coding for the precursor of lingual lipase. The precursor protein is for example prelingual lipase protein, or a fusion protein comprising lingual lipase and a heterologous protein. A pharmaceutical composition in unit dosage or liquid form is described.

EP 0 131 418 A1

Croydon Printing Company Ltd.

PROTEINS, PHARMACEUTICAL COMPOSITIONS, GENES, VECTORS, HOST ORGANISMS AND PROCESSES FOR THEIR PRODUCTION

This invention relates to proteins, pharmaceutical compositions, genes, vectors, host organisms and processes for their production.

The lipolysis of dietary fat is an important feature of the digestive systems of higher animals. The digestive process is made possible by enzyme catalysed hydrolysis of triglycerides to produce a mixture of monoglycerides, diglycerides, glycerol and free fatty acids as the fats pass through the digestive tract. The hydrolysis products are able to pass through the epithelial membrane of mucosal cells lining the gut. Once absorbed they are used to resynthesise triglycerides which are incorporated in chylomicrons. Chylomicrons are transported by the lymph system away from the site of absorption. The enzymes carrying out triglyceride hydrolysis are termed lipases and are secreted into the gastrointestinal tract (Desnuelle, P (1972) The Enzymes Vol. VII, 3rd Edition. Acad. Press New York and London p 575-616, and Verger, R. (1980) Methods in Enzymology 64, 340-392).

An enzyme involved in triglyceride hydrolysis is pancreatic lipase (EC 3.1.1.3). The pig is a convenient source of enzyme and pig pancreatic lipase has been extensively studied. It is present at approximately 2.5% of the total proteins in pig pancreatic juice, and has been purified to homogeneity (Verger, R. et al (1969) Biochim Biophys Acta 188, 272-282). The complete amino acid sequence of the enzyme has been determined (De Caro, J. et al (1981) Biochim Biophys Acta 671, 129-138). The enzyme comprises a protein portion of 449 amino acids (MW 49859) with a carbohydrate portion (MW about 2000) attached to an Asn residue at position 166 in the amino acid sequence.   The total molecular weight of the enzyme is therefore approximately 52000.   The catalytic activity of pancreatic lipase is complex since there exists a phase separation between the soluble enzyme and the insoluble triglyceride substrate.   In order for the enzyme to interact with the substrate an coenzyme known as colipase is necessary.   Colipase is a low molecular weight protein which adsorbs to the solution/lipid interface and then acts as an anchor for lipase, allowing interaction between the enzyme and its lipid substrate.

Pancreatic lipase may be assayed by a variety of techniques (see Desnuelle and Verger as above) involving the measurement of the disappearance of the triglyceride or the appearance of free fatty acid or glycerol. Radioactive labelling, proton release during hydrolysis, and the effect of lipase on the physical properties of a lipid monolayer may also be used to assay lipase activity. In all cases pancreatic lipase is optimally active in the neutral-alkaline pH range (i.e. pH7-pH9) (see Verger et al as above).   The enzyme is highly sensitive to acid pH and is rapidly inactivated at low pH.

A number of lipid malabsorbtion illnesses of the human body are characterised by reduced levels of pancreatic  lipase secretion.

About eighty percent of individuals suffering from cystic fibrosis suffer from an insufficient pancreatic function.   Pancreatic lipase insufficiency manifests itself shortly after birth and continues throughout the lifetime of the patient.

Pancreatitis is a condition in which the action of the pancreas is impaired.   Pancreatitis often develops in chronic alcoholics who, as a result suffer from malabsorption of fats and consequent malnutrition.

- 3 -    **0131418**

A developing foetus is dependent upon high carbohydrate nutrition, and has a poorly developed pancreatic function producing low levels of pancreatic lipase.    At birth the high carbohydrate nutrition  of the foetal period is replaced by a high fat diet as the infant begins to take its mothers milk.    Fats account for about half an infants calorie input.    The pancreatic function, even in infants that are carried to full term, is not fully productive and infants, especially those born prematurely, may suffer from inadequate fat digestion leading to appreciable steatorrhea (passage of undigested fat in the faeces) and to a resulting loss of energy.

The present treatment of patients suffering from a deficiency of pancreatic lipase is the oral administration of very large doses of a crude preparation of pig pancreas enzyme.    Pancreatic lipase is inactivated by the low pH conditions in the stomach, with the result that orally administered pancreatic lipase is substantially in activated on passage through the stomach to the gut.    The large doses administered are inadequate for most patients, are impure and are unpalatable.    Certain tablets have been formulated which pass through the acid regions of the stomach and discharge the enzyme only in the relatively alkaline environment of the jejunum (Gow, R. et al (1981) The Lancet Vol. II 8255, 1070-1074).    However, many patients suffering from pancreatic disorders have an abnormally acid jejunum and such tablets may fail to discharge the enzyme and may therefore be ineffective.

There is a great need for a preparation of a lipase which may be orally administered to patients suffering from a deficiency of pancreatic lipase.

The lingual serous (Von Ebner) gland which is situated in the dorsal posterior of the tongue, has been found to secrete a potent lipase known as lingual lipase.    The enzyme has been detected in the rat (Hamosh, M. and Scour, R.O. (1973) J. Clin. Invest. 52, 88-95) and in humans (Hamosh , M. and Burns, W.A. (1977) Lab. Invest. 37, 603-608). The enzyme is swallowed with saliva and enters the stomach where it effects a partial lipid digestion. The enzyme has been partially purified from the Von Ebner's gland of rats (Fields, R.B. et al, (1983) J. Biol. Chem. 258, 14563-14569, Hamosh M. et al (1979) J. Biol. Chem. 254, 12121-12125). Lingual lipase hydrolyses triglycerides both in vivo and in vitro to diglycerides, monoglycerides and free fatty acids (Hamosh, M. (1984), "The Lipases", Borgstrom,.B. and Brockman, H.O., Eds., Elsevier, Amsterdam). A major difference between lingual lipase and pancreatic lipase is that lingual lipase is stable in acid environments such as are found in the stomach, especially after feeding. It has been demonstrated that lingual lipase is capable of hydrolysing triglycerides at pHs in the range from 2 to 7. Lingual lipase is only available in extremely small quantities from natural sources. For example a preparation from 16 rats. produced approximately 1.5 mg of impure enzyme (Field et al (1983) 258, 14563-14569). The purification of an acid stable lipase from human stomach aspirates has been reported by Tiruppathi, C. et al (1982) Biochim. et Biphys. Acta 712 692-697).

All the work reported above on lingual lipase has been exclusively of an academic nature, and no suggestion has been made of using lingual lipase for the treatment of lipase deficiency. We believe that lingual lipase can be so used, but it is only economic to do so if lingual lipase can be produced on a large scale and at relatively little expense. It is clearly not possible to do this by extraction from animal or human tongues.

We provide lingual lipase in such commercially worthwhile amounts by producing it, in accordance with the invention, using recombinant DNA techniques.

According to a first aspect of the present invention we provide a lingual lipase protein for use in the treatment of lipase deficiency.

As used herein the term "lingual lipase protein" denotes an authentic mammalian lingual lipase or an authentic mammalian lingual lipase modified or substituted to provide a functionally equivalent protein. The lingual lipase protein may, for example, comprise a mammalian lingual lipase with an N-terminal methionine amino acid residue (a methionine-lingual lipase protein). Preferably the lingual lipase protein is a human or rat lingual lipase protein.

The lingual lipase protein is advantageously produced by a recombinant DNA technique.

In a second aspect of the invention we provide a process for the production of a methionine-lingual lipase protein comprising producing the protein in a host organism transformed with a vector including a gene coding for the methionine-lingual lipase protein. Preferably the host organism is a bacterium (for example E.coli) or a yeast (for example Saccharomyces cerevisiae).

In a third aspect of the invention we provide a process for the production of a lingual lipase protein comprising producing a lingual lipase precursor protein in a host organism transformed with a vector including a gene coding for the precursor protein and cleaving the precursor protein to produce the lingual lipase protein.

Preferably the lingual lipase precursor protein is a prelingual lipase protein and the host organism is a host organism capable of cleaving the prelingual lipase protein to produce the lingual lipase protein. Most preferably the host organism cleaves the prelingual lipase and may export the lingual lipase protein to the culture medium.

In an alternative form of the third aspect of the invention the precursor lingual lipase protein is a fusion protein comprising a heterologous protein and a lingual lipase protein. The heterologous protein may be all or a part of a protein capable of production, desirably at a high level, in the host organism. Suitable such proteins include β-galactosidase and the product of the trpE gene. The fusion protein preferably includes a site susceptible to selective chemical or enzymic cleavage between the heterologous protein and the lingual lipase protein. The heterologous protein may be a yeast signal sequence and the host organism may be yeast. In this preferred embodiment the yeast host organism advantageously cleaves the fusion protein to produce a mature lingual lipase protein.

In a fourth aspect of the invention we provide a prelingual lipase protein.

In a fifth aspect of the invention we provide a methionine-lingual lipase protein.

In a sixth aspect of the invention we provide a fusion protein comprising a heterologous protein and a lingual lipase protein.

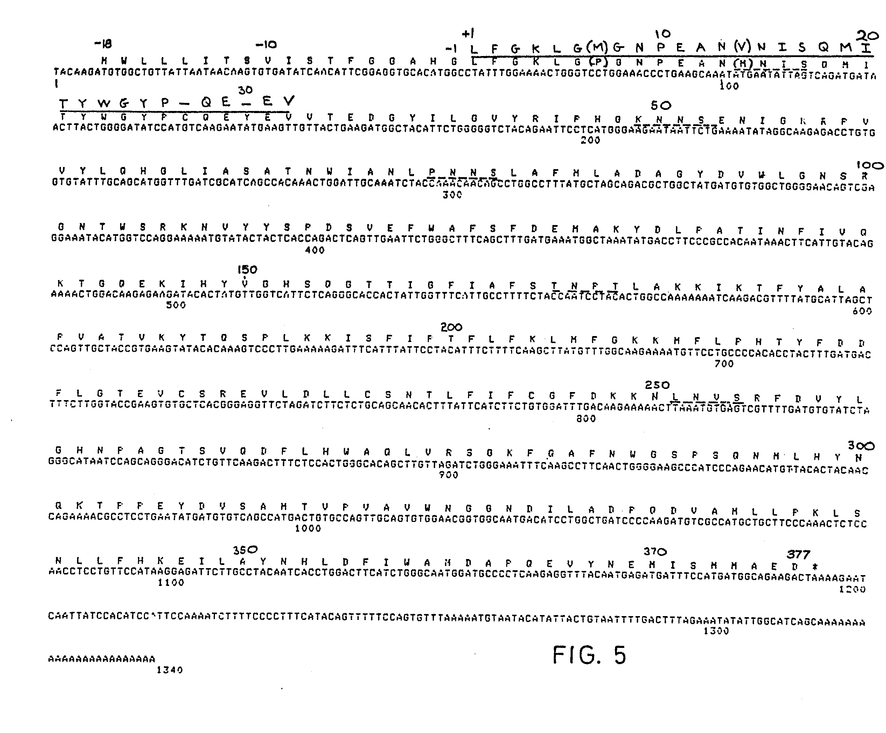

In a seventh aspect of the invention we provide a gene coding for at least the amino acid sequence of a lingual lipase protein. Preferably the gene codes for a protein of the fourth, fifth or sixth aspect of the invention. We further provide a gene coding for at least the amino acid of rat lingual lipase as shown in Figure 5 of the accompanying drawings.

In an eighth aspect of the invention we provide a vector including a gene of the seventh aspect of the invention. The vector is adapted for use in a given host organism by the provision of suitable selectable markers, promoters and other control regions as appropriate.

In a ninth aspect of the invention we provide a host organism transformed with a vector according to the eighth aspect of the invention. The host organism may be any organism which may be transformed by a vector including a gene coding for a lingual lipase protein such that expression of the gene occurs. Suitable such host organisms include bacteria (for example E.coli), yeasts (for example Saccharomyces cerevisiae) and mammalian cells in tissue culture. Preferably, where the host organism is a bacterium or a yeast the vector includes a gene coding for methionine-lingual lipase or a fusion protein, and when the host organism is a mammalian cell in tissue culture the vector preferably includes a gene coding for prelingual lipase.

In a tenth aspect of the invention we provide an antibody having specificity for an antigenic determinant of a lingual lipase protein. The antibody may be a polyclonal or monoclonal antibody, but is preferably a monoclonal antibody. The antibody may be labelled with a detectable marker, for example a radioactive isotope, for use in immunoassay.

In an eleventh aspect of the invention we provide a pharmaceutical composition comprising a lingual lipase protein and a pharmaceutically acceptable excipient. Preferably the lipase protein is a human or rat lingual lipase produced by a process of the second or third aspect of the invention. The pharmaceutical composition is provided for use in the treatment of lipase deficiency. Preferably the composition is formulated for oral administration. The composition may be in a unit dosage form, for example as a tablet, capsule or dragee.

To produce a unit dosage form, the lingual lipase, in a suitable form, may be mixed with a solid pulverulent non-pharmaceutically active carrier such as lactose, saccharose, sorbitol, mannitol, starch, cellulose derivatives or gelatine or any other such excipient. A lubricant such as magnesium stearate, calcium stearate or polyethylene glycol wax may be added. The resulting composition is compressed to form a unit dosage form. The unit dosage form may be coated with a concentrated sugar solution which may contain additives  such as talc, titanium dioxide, gelatine or gum  arabic.   The unit dosage form may be coated with lacquer. Dyestuffs may be added to the coating to facilitate identification of the unit dosage form. Soft or hard capsules may be used to encapsulate lingual lipase as a liquid or solid preparation.

Alternatively, the lingual lipase may be formulated in a liquid form. To produce a liquid form of preparation the lingual lipase in a suitable form may be added to a liquid carrier. The carrier may be, for example, a syrup or a suspension. The liquid form may contain colouring compounds, flavouring compounds, sweetening compounds, and/or thickening compounds.

In a further aspect of the invention we provide a process for the production of a pharmaceutical composition comprising bringing a lingual lipase protein into association with a pharmaceutically acceptable carrier. In a yet further aspect of the invention we provide a method for the treatment of lipase deficiency comprising administering an effective amount of a lingual lipase protein.

In the following description a protocol is described for the production of lingual lipase using recombinant DNA technology, in which reference is made to the following drawings:-

Figure 1 - shows an SDS polyacrylamide gel of purified rat lingual lipase,

Figure 2 - shows an SDS polyacrylamide gel of the digestion of purified rat lingual lipase with Endonuclease H,

Figure 3 - shows a restriction site map of plasmid pLL10,

Figure 4 - shows a restriction map of rat LL DNA (4 a)), the alignment of the rat LL protein with the DNA sequence (4 b)), and the DNA sequence (with related amino acids) of the β-galactosidase/ prelingual lipase gene junction (4c)),

Figure 5 - shows the DNA sequence of the rat
               prelingual lipase gene, and
Figure 6 - shows an SDS polyacrylamide gel showing
               the production of a prelingual lipase/
               β-galactosidase fusion protein by pLL10.

The strategy used was to first purify lingual lipase from rat tongue (a more plentiful and convenient source of the enzyme than human tongue).  The purified rat enzyme was subjected to N-terminal amino acid sequencing, structural characterisation and a polyclonal antiserum was raised.  The rat enzyme was cloned by screening an expressed cDNA library derived from rat von Ebner's gland (a more plentiful source of mRNA than human tongue tissue) with the rat polyclonal antiserum. The complete nucleic acid/protein sequence of rat lingual lipase was determined.  The following description describes how the rat  enzyme clone is expressed in an appropriate microorganism or animal cell in tissue culture and a recombinant rat lingual lipase is produced. Also described is the method by which the human lingual lipase gene is cloned, expressed and a recombinant human lingual lipase produced.

1. Purification of Lingual Lipase Enzyme from the
   von Ebner's Gland of Rat

Complete tongues were removed from freshly killed rats and the von Ebner's gland was removed by dissection. Approximately 600 glands were homogenised and delipidated by extraction with organic solvents and an impure vacuum dried powder extract produced as described by Verger, R. et al.(1969) Biochim. Biophys. Acta. 188, 272-282.   An impure lipase rich cream was produced essentially as decribed by Verger (1969) and Melius, P. et al (1965) Biochim. Biophys. Acta. 105 60 with certain modifications, i.e. the initial powder was suspended in 2% (v/v) Triton X100, 0.15M NaCl together with proteinase inhibitors and clarified by centrifugation before extraction with butanol-ammonium sulphate. The cream was extracted with 50mM sodium acetate pH5 (containing proteinase inhibitors and Triton X100). The suspension was dialysed against the sample buffer minus Triton X100 and clarified by centrifugation to produce a crude extract. This crude extract was chromatographed on CM-Sepharose CL6B and eluted with a 0-0.7M NaCl gradient in 50mM sodium acetate pH5.

A single peak of lipase activity eluted at approximately 0.27M NaCl which was pooled, concentrated and subjected to gel filtration on Sephadex G150 in 50mM sodium acetate. A single peak of lipase activity eluted at approximately 1.7 times the void volume. Samples of fractions containing lipase activity were analysed by SDS-PAGE electrophoresis and the preparation stored at $-20°C$.

The lipolytic activity of the enzyme was measured throughout the purification procedure. Lingual lipase was measured in the acid pH range using a variety of triglyceride substrates by the technique of lipid monolayer spreading as described by Verger R. and Patton, F. (1982) Chemistry and Physics of Lipids 30, 189-227, the use of a potentiometric technique (Verger et al (1969), or alternatively a radioassay using $[^3H]$ triolein substrate as described by Hamosh et al (1979). J. Biol. Chem. 254, 12121-12125. The radioassay was modified by inclusion of emulsified anhydrous glycerol as described by Patton et al (1982) Biochim. Biophys. Acta 712, 400-407 and extraction of free fatty acids in 0.1 N glycine pH12.5 and organic phase described by Belfrage and Vaughan (1969) J. Lipid. Res. 10, 341.

The initial extraction procedures described above were satisfactory in that they routinely recovered greater than 50% of the initial lipase activity in the delipidated extracted cream.   However, the chromatographic steps were subject to a low final lipase activity yields (approx. 10%) and lingual lipase of variable purity between batches.

When analysed by SDS-PAGE electrophoresis individual preparations of the final product contained between 10-90% of a single polypeptide chain migrating with an apparent molecular weight of 52,000 (close to the estimated molecular weight of rat lingual lipase (Fields, R.B. et al (1983)).

The purification process ..was therefore improved by replacement of the above chromatographic steps with a single immunopurification procedure : utilising a polyclonal rabbit anti-rat lingual lipase antibody coupled to Sepharose.

Rat lingual lipase was partially purified by chromatography on CM Sepharose CL6B and gel filtration on Sephadex G150 (as described above).   100 μg of a preparation consisting of approximately 90% protein by weight with a molecular weight of approximately 52,000 was taken up in 1 ml complete Freund's adjuvant and injected into a rabbit. After 14 days the innoculation was repeated using incomplete Freund's adjuvant.   The rabbit was bled to produce antiserum after 28-30 days and at 30 day intervals subsequently.   The titre of the antiserum was determined by standard immunological procedures.

Immunoglobulins were precipitated from high titre rabbit anti rat lingual lipase antiserum with solid sodium sulphate (18% w/v), redissolved in 0.1M sodium bicarbonate pH9 and dialysed extensively against the same buffer.   The immunoglobulins were coupled to cyanogen bromide activated Sepharose (Pharmacia) according to the manufacturer's instructions.   The affinity adsorbent was blocked with 1M ethanolamine (adjusted to pH8) for 2 hours at room temperature, washed with bicarbonate and acetate buffers and stored in 0.02% (w/v) sodium azide at 4°C until use.

Proteins solubilised from the lipase rich cream (as described above) were applied to a column packed with rat anti lingual lipase-Sepharose. The column was washed with phosphate buffered saline and 1M NaCl in phosphate buffered saline. Bound protein was eluted in 0.1M glycine HCl pH2.3 and dialysed against 50mM ammonium acetate pH5, freeze dried and stored as a powder at -20°C. This procedure produced pure rat lingual lipase with a molecular weight of approximately 52,000 as judged by SDS PAGE electrophoresis. The use of this immunoabsorbent column greatly improved the yield, purity and reproducibility of the purification procedure. Approximately 100 μg of electrophoretically pure rat lingual lipase was obtained per rat tongue with a specific activity of approximately 500 units/mg (unit = micromoles of fatty acid formed per min. at 37°C).

## 2. Characterisation of Authentic Rat Lingual Lipase

### 2.1  Determination of Molecular Weight of Rat Lingual Lipase

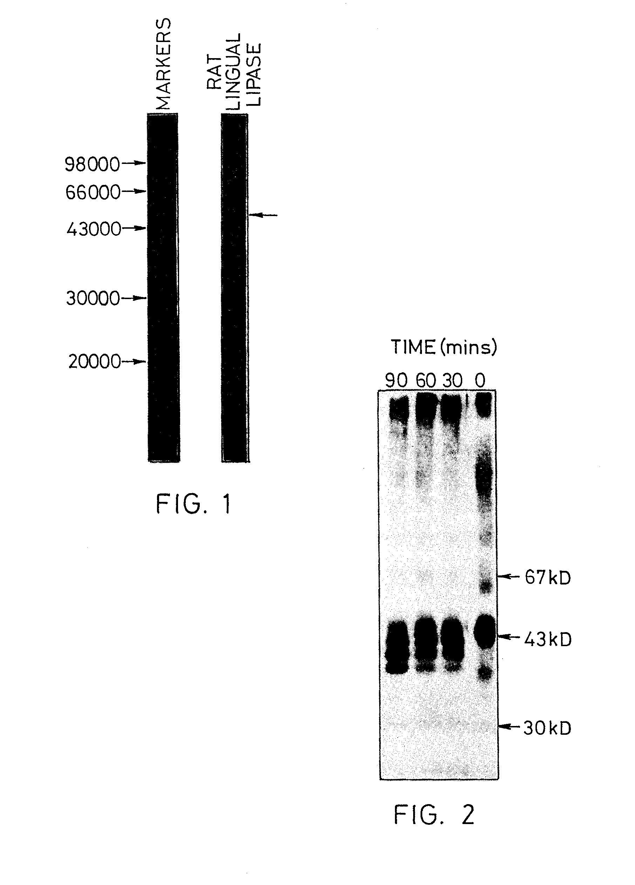

Rat lingual lipase, purified to homogeneity by immunoaffinity chromatography, was subjected to electrophoresis in SDS polyacrylamide gels (Laemmli (1970) Nature 227, 680-685) in the presence of several proteins of known molecular weight. 5 μg of rat lingual lipase was applied into a 7.5% (w/v) acrylamide gel. The proteins were visualised with Coomassie Blue and the resulting gels are shown in Figure 1. An arrow marks the position of migration of the enzyme with respect to the marker proteins, for which approximate molecular weights are shown. The enzyme migrated as a single band with an apparent molecular weight of 52,000. Gel filtration of impure lingual lipase on Sephadex G150 resulted in a calculated molecular weight in approximate agreement with that obtained by polyacrylamide gel electrophoresis. A molecular weight of 51,000 has been estimated by Field et al (1983) using gel filtration on Sephadex G200. It is therefore concluded that the purified rat lingual lipase is active as a monomer of approximately 52,000 molecular weight.

### 2.2 Determination of the Presence of Glycosylation in Rat Lingual Lipase

The presence of asparagine linked N-glycosylation was established by digestion of purified rat lingual lipase with Endoglycosidase H (Endo-β-N-acetylglucosamini-dase H) from streptomyces plicatus. A 1 mg/ml solution of rat lingual lipase in 50 mM sodium acetate pH5.5, 1 mM phenyl methyl sulphonyl fluoride, 10 µM pepstatin A containing 50 units/ml Endoglycosidase H was incubated at 37°C. Alternatively, rat lingual lipase was boiled in 0.4% (w/v) SDS and diluted to 0.1% (w/v) SDS before incubation as above. In both cases the rat lingual lipase digestion products were separated on SDS-PAGE (7.5% (w/v) acrylamide) and visualised by Western Blot analysis (Burnette, W.N. (1981) Analyt. Biochem. 112, 195-203) using rabbit anti rat lingual lipase antiserum. Digestion of rat lingual lipase (MW approx. 52,000) resulted in the generation of a series of lower molecular weight forms with a minimum molecular weight of approximately 41,000 (Figure 2). Endoglycosidase H digestion results in the removal of N linked carbohydrate moieties from glycoproteins containing these residues. This cleavage produces an apparent lowering of the molecular weight of the deglycosylated protein. This lowering of molecular weight may be visualised by increased mobility of the deglycosylated protein on SDS-PAGE. That Endoglycosidase treatment of rat lingual lipase is an apparent decrease of molecular weight from 52,000 to 41,000 indicates that approximately 20% of the enzyme (by weight) is composed of carbohydrate.

## 2.3 N-Terminal Amino Acid Sequence and total Amino Acid Composition of Rat Lingual Lipase

The N-terminal amino acid sequence of purified rat lingual lipase was determined by the method of Smith, M.A. et al (1982) 207, 253-260.

N-Terminal Amino Acid Sequence of Rat Lingual Lipase

1                                                    10 Leu Phe Gly Lys Leu Gly Met Gly Asn Pro Glu Ala Asn Val Asn Ile Ser Gln 20                                          30 Met Ile Thr Tyr Trp Gly Tyr Pro - Gln Glu - Glu Val (a hyphen indicates residue not determined).

A partial amino acid composition was determined by the method of Campbell et al (1979) Biochem. J. 177, 531-540.

## TABLE 1

### Partial Amino Acid Composition of Rat Lingual Lipase

| Residue | nMoles Amino Acid Eluted | Total Number of Amino Acids Predicted from DNA Sequence |
|---|---|---|
| Asp + Asn | 5.74 (total) | 19 + 27 |
| Thr | 2.18 | 20 |
| Ser | 2.42 | 23 |
| Glu+ Gln | 4.13 (total) | 15 + 14 |
| Pro | 2.68 | 21 |
| Gly | 3.16 | 25 |
| Ala | 3.05 | 26 |
| Val | 2.66 | 24 |
| Met | 1.31 | 13 |
| Ile | 2.10 | 20 |
| Leu | 3.88 | 34 |
| Tyr | 1.90 | 19 |
| Phe | 2.66 | 25 |
| Lys | 2.46 | 22 |
| His | 1.28 | 10 |
| Arg | 1.14 | 7 |

Cys and Trp were not determined.

3. Cloning of the Rat Lingual Lipase Gene

The von Ebner's glands of newly sacrificed rats were dissected from the back of the tongues and were frozen in liquid nitrogen (see Hamosh, M. et al (1979) J. Biol. Chem. 254, 12121-12125). The glands were crushed under liquid nitrogen and the total mRNA was extracted using the guanidinium isothiocyanate procedure (Maniatis et al (1982) Molecular Cloning: A Laboratory Manual. Cold Spring Harbor Laboratory). Polyadenylated RNA was isolated by oligo-dT cellulose chromatography (Harris, T.J.R. and Wildly, P. (1975) J. Gen. Virol. 28, 299-312) and mRNA integrity was monitored by in vitro translation in a reticulocyte lysate (Pelham, H. and Jackson, R. (1976) Eur. J. Biochem 67, 247-256). Single stranded cDNA was synthesised essentially as described in Retzel, E.R. et al (1980) Biochemistry 19, 513-518, and was converted to double stranded cDNA by the procedure described in Rose, J.K. and Gallione, C.T. (1981) J. Gen. Virol. 39, 519-528). The products of the procedure were treated with $S_1$ nuclease to remove regions remaining single stranded and were treated with $T_4$ polymerase to produce blunt ends. (Maniatis et al see above).

cDNA produced by the above procedure was separated into four equal fractions. Fraction 1 was digested with Sau 3A and Fractions 2, 3 and 4 were ligated to Bcl I, Bam HI and Bgl II linkers respectively (Maniatis et al see above). The linked DNA was digested with Bcl I, Bam HI and Bgl II and separated from the excess linkers by Biogel A50 chromatography. cDNA from each of the fractions was inserted by cohesive end ligation (Maniatis et al see above) into the Bam HI site of a series of expression vectors designed to produce either β-galactosidase or trpE fusion polypeptides in all three reading frames. The β-galactosidase fusion vectors have been described by Ruther and Muller-Hill (1983) EMBO J. 2, 1791-1794 and the trpE fusion vectors are described in our copending international patent application PCT/GB 83/00172, published as WO 84/00380 on 2nd February, 1984.

The ligated DNA was transformed into E.coli, DH1 cells by the procedure of Hanahan, D. (J. Mol Biol. 166 p557-580 (1983). The resulting recombinant colonies were replicated onto nitrocellulose filters and then screened as follows for those producing protein with rat lingual lipase antigenic determinants. The colonies were lysed on the filter with 9M urea in 10mM Tris Hcl pH7.5, 1mM EDTA in a chloroform saturated atmosphere. Cell debris was removed from the filters by washing extensively in buffer containing 160 mM NaCl, 10mM Tris HCl pH7.5. DNase at 5 $\mu$g/ml was used to remove DNA. After further washing non-specific binding sites on the filters were blocked by incubating with washing buffer containing 0.5% casein.

The filters were incubated colony side down in the washing buffer containing in addition 0.1% Triton X100, 0.1% SDS, 1mM EDTA and about 2 $\mu$g/ml IgG (purified from rabbit anti rat lingual lipase serum, see above). The IgG preparation had previously been preabsorbed against a total protein extract from E.coli DH1. Thorough washing was then undertaken to remove unbound IgG from the filters which were subsequently incubated with 1$\mu$Ci/ml Protein A labelled with $^{125}$I. The filters were finally washed with the SDS and Triton X100 containing buffer described above and subjected to autoradiography. Colonies producing protein with rat lingual lipase antigenic determinants were analysed further for the presence of cloned rat lingual lipase sequences by the technique of hybrid selection (Ricciardi, R.P. et al (1979) Proc. Natl. Acad. Sci. (USA) 76, 4927-4931.

Plasmid preparations from candidate colonies were also screened by partial DNA sequence analysis of cloned DNA fragments. DNA fragments were cloned for sequencing into the M13 vectors mp8 and mp9 (J. Messing and J. Vieira (1982) Gene 19, 269-276). The sequences were determined by the dideoxy sequencing method (Sanger F.S. et al (1977) PNAS 74, 5463; A.J.H. Smith (1980) Methods in Enzymology, Academic Press. 65, 500) using a synthetic single-stranded universal primer which hybridised to a region just 3' to the cloned part.

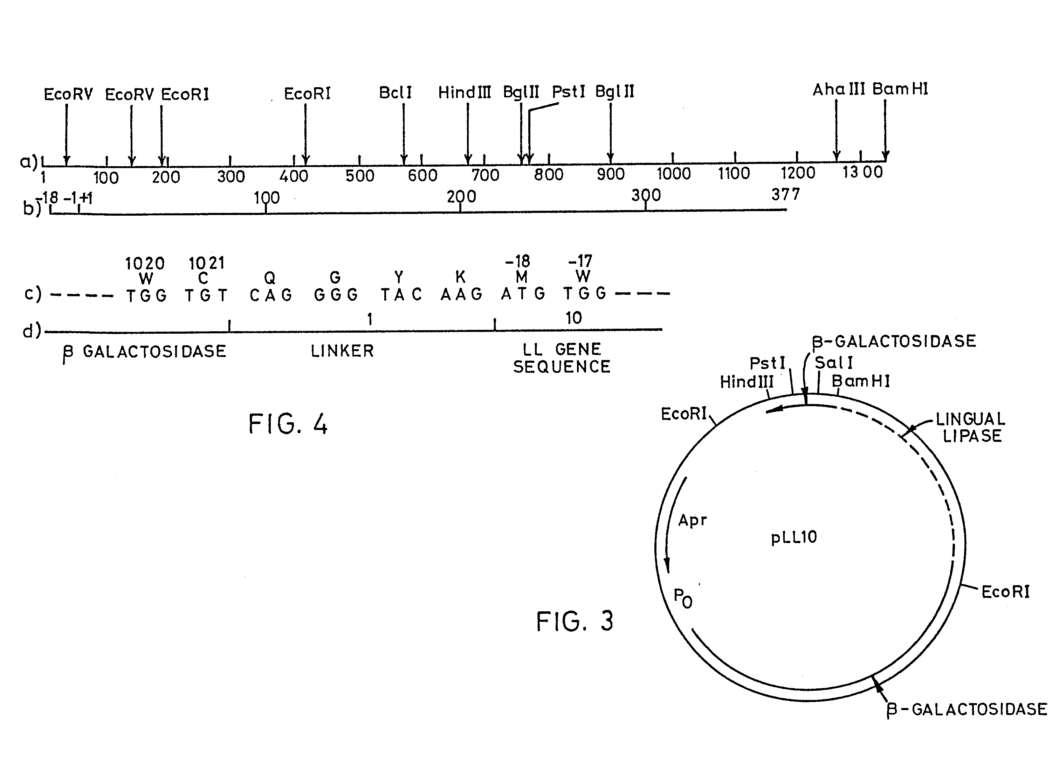

A plasmid, designated pLL10, (Figure 3 ) was identified, which contains a DNA insert of approximately 1350 bases and was therefore capable of encoding the entire amino acid sequence of rat lingual lipase.

The plasmid pLL10 is derived from plasmid pUR292 (Ruther and Muller-Hill see above). The cloned pre-lingual lipase (broken line) is inserted into the Bam HI site of pUR292.

A restriction endonuclease map of pLL10 was constructed (Figure 4 a)) and the complete DNA sequence of the insert determined (Figure 5).

In Figure 4 a) the position of several restriction endonuclease cleavage sites in the rat lingual lipase DNA sequence are shown by arrows. Numbers below the line relate to the base numbers given in Figure 5. Figure 4 b) shows the alignment of the rat lingual lipase protein with the DNA sequence. Numbers above the line relate to amino acid numbers given in Figure 5. Position +1 is the N terminal residue of mature lingual lipase. Position 377 is the C terminal amino acid of the mature protein. Amino acids -1 to -18 represent the putative rat lingual lipase signal sequence starting with a met at position -18. Figure 4 c) shows the β-galactosidase/pre lingual lipase junction sequence. In plasmid pLL10 the coding region of pre rat lingual lipase is fused via a 4 amino acid encoding linker to residue 1021 of β-galactosidase. The Figure shows the DNA and derived amino acid sequences at the junction of the β-galactosidase and lingual lipase genes. Letters above the DNA sequence indicate the derived amino acid sequence in this region. Numbers above the amino acid code letters indicate the amino acid number in the β-galactosidase or lingual lipase gene. The lower line indicates the origins of the sequence.

In Figure 5 the DNA sequence of the coding strand of the pre lingual lipase gene is shown.    Numbers below the DNA sequence represent the base number.    Base 1 is arbitarily placed 6 nucleotides upstream from the ATG codon of the putative initiating met residue.    "*" indicates the stop codon TAA which is followed by a 3' untranslated region and poly A sequence.    Letters immediately above the bases represent the derived amino acid sequence, using the conventional single letter amino acid code (i.e. A=alanine, R=arginine, N=asparagine, D=aspartic acid, C=cysteine, E=glutamic acid, Q=glutamine, G=glycine, H=histidine, I=isoleucine, L=leucine, K=lysine, M=methionine, F=phenylalanine, P=proline, S=serine, T= threonine, W=tryptophan, Y=tyrosine and V=valine).    Underlined letters above the derived amino acid sequence represent the N-terminal amino acid sequence obtained directly from purified rat lingual lipase.    Amino acids in brackets represent discrepancies between the amino acid sequence derived by DNA sequencing and that obtained amino acid sequencing of purified lingual lipase. Spaces in the directly obtained amino sequence represent undetermined amino acids.    Amino acids -18 to -1 represent a putative signal sequence and +1 to 377, the amino acid sequence of the mature protein. Broken underlining indicates the potential glycosylation sequence around an asparagine residue.

The amino acid sequence predicted from the DNA sequence indicates that mature lingual lipase consists of a 377 amino acid protein (terminating with a stop codon) (Figure 5).    The predicted molecular weight of this mature protein is 42,564 which is in close agreement with the molecular weight determined for the deglycosylated enzyme by SDS PAGE.    The total amino acid composition of the mature enzyme predicted from the DNA sequence is compared with that obtained directly from the isolated protein in Table 1.    Mature lingual lipase contains five potential sites for glycosylation ( of the general form X Asn X Thr or Ser). Rat lingual lipase is 72 amino acids shorter than porcine pancreatic lipase and bears little sequence homology or amino acid composition similarity to this enzyme. However, close homology does exist between rat lingual lipase and porcine pancreatic lipase in the region of the essential serine-152 of porcine pancreatic lipase. This serine is thought to participate in the interfacial fixation of pancreatic lipase to lipid (Guidoni, A. et al 1981, Biochim. Biophys. Acta. 660, 148-150) and reacts with micellar diethyl-p-nitrophenyl phosphate (Rouard, M. et al 1978, Biochim. Biophys. Acta. 530, 227-235). It is present in the sequence:

> 152
> Gly - His - Ser - Leu - Gly    Porcine Pancreatic Lipase and in a closely equivalent position in the primary amino acid sequence:

> 153
> Gly - His - Ser - Gln - Gly    Rat Lingual Lipase

Another point of similarity close to this essential serine residue is the single glycosylation position in porcine pancreatic lipase (Asn-166) which appears to be present as Asn-166 in rat lingual lipase.

4. Expression of Rat Prelingual Lipase in E.coli as a Fusion Protein with β galactosidase

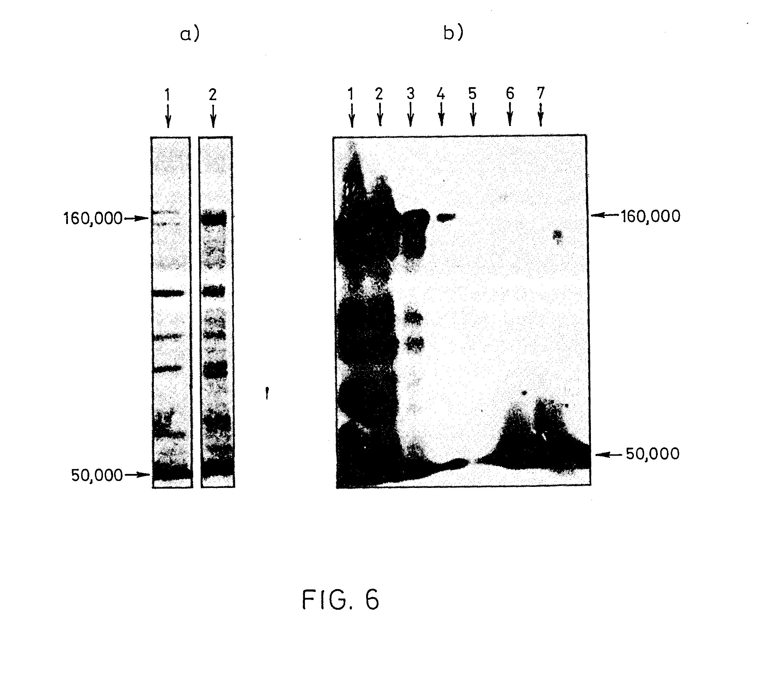

Overnight cultures of E.coli containing pUR292 and pLL10 plasmids were grown in L-broth plus ampicillin at 37°C. 1 ml samples were transferred to 100 mls L-broth plus ampicillin and grown at 37°C for 5 hours to an $OD_{600}$ of approximately 1.0. The culture was cooled on ice and cells collected by centrifugation at 4°C. Samples of cells equivalent to 1 to 0.01 optical density unit (at 600 nanometres) were boiled in SDS-mercaptoethanol sample buffer, applied to a 5% polyacrylamide SDS gel and stained with Coomassie Blue (Figure 6a). Lane 1 shows high molecular weight E.coli proteins from cells containing pUR292. Lane 2 shows total proteins from cells containing pLL10. A large band comprising 5-10% of total E.coli protein is visible in Lane 2 migrating with an apparent molecular weight of approximately 160,000 (close to the $\beta$ subunits of E.coli RNA polymerase (approximately 160,000 MW). This band is not visible in cells containing pUR292. The molecular weight of the lingual lipase/$\beta$-galactosidase fusion protein (predicted from the DNA sequence of lingual lipase and the known molecular weight of $\beta$-galactosidase) is approximately 160,000.

The protein expressed from pLL10 therefore has a similar molecular weight to that predicted for the fusion protein.

This was confirmed by Western blot analysis of a similar polyacrylamide gel (Figure 6b). Lanes 1, 2, 3, 4 contain total proteins extracted from 1, 0.5, 01 and 0.01 $OD_{600}$ unit respectively of E.coli/pLL10. Lane 5 contains total proteins from 1 $OD_{600}$ unit of E.coli/pUR292. Lanes 6 and 7 contain approximately 1 $\mu$g of purified authentic rat lingual lipase. As can be seen from Lanes 1 - 4 a major protein is detected with a molecular weight of approximately 160,000, together with several proteins of lower molecular weight. No immunoreactive proteins are detected in an extract prepared from pUR292 (Lane 5). Samples of rat lingual lipase purified from rat tongue also react with the antiserum (Lane 6,7). It is therefor concluded that the rat lingual lipase has been expressed at high levels in E.coli as a fusion protein with $\beta$-galactosidase.

## 5. Expression of Rat Lingual Lipase in (1) E.coli, (2) Yeast, (3) Tissue Cultured Animal Cells

### 5.1 E.coli

Plasmid vectors for the expression of mature rat lingual lipase are constructed based on pCT54 (Emtage, J.S. et al PNAS U.S.A. (1983), 80 3671-3675). These vectors contain the trp promoter, optimised Shine-Delgarno ATG base sequence preferably adjacent to the (Met) Lingual lipase gene. Appropriate construction of these vectors will also express pre-lingual lipase or a fusion protein of trpE gene product and lingual lipase or pre-lingual lipase. E.coli containing these plasmids when grown under optimum conditions for lipase expression produce met-lingual lipase, pre-lingual lipase or a fusion protein at levels up to 10% of total cellular protein. The presence of the enzyme in crude extracts of total cells is confirmed and quantified by gel scanning and immunoprecipitation. (Emtage et al (1983) Proc. Natl. Acad. Sci. (U.S.), 80, 3671-3675).

The soluble protein fraction from crude E.coli extracts is assayed for lipolytic enzyme activity by the methods described above. A part or whole of the enzyme expressed in E.coli may be in an insoluble inactive form and therefore not detected in the above assay. In such circumstances the enzyme is solubilised and activated prior to assay and purification as described in relation to chymosin production in our co-pending international patent application PCT/GB 83/00152 (published as WO 83/ 04418) and in published British patent application GB 2100737A. Having obtained soluble, active, partially purified enzyme by the above techniques the enzyme is further purified as described above, or using other standard protein purification techniques.

## 5.2 Yeast

Plasmid vectors for the expression of mature rat lingual lipase are constructed based on vectors described in co-pending published European patent application EP-A2-0073635. These vectors contain the yeast PGK promoter and PGK gene 3' end flanking sequences sandwiching a (Met) lingual lipase, pre lingual lipase or a fusion protein between PGK and met lingual lipase or pre lingual lipase. Yeast containing these plasmids when grown under optimum conditions for lipase expression produce met lingual lipase, pre lingual lipase or a fusion protein at levels up to 10% of total cellular protein. Expressed lipase are quantified, assayed and purified as described above.

## 5.3 Tissue Cultured Animal Cells

Plasmid vectors for the expression of rat pre lingual lipase will be constructed based on vectors described by Pavlakis, G.N. and Hamer, D.H. (1983) Proc. Natl. Acad. Sci. U.S.A. 80, 397-401. These vectors contain metallothionine gene promoters and express pre lingual lipase. The enzyme produced in this system is secreted through the cellular membrane and can be assayed in and purified from the tissue culture medium as described above.

## 6. Human Lingual Lipase

The procedures outlined above describe the cloning, expression and purification of rat lingual lipase. Similar procedures are carried out to obtain the analogous enzyme from a human source.

A cDNA clone of human lingual lipase is obtained starting from human tongue mRNA. RNA is prepared from a sample of human tongue by extraction with guanidinum isothiocyanate (Chirgwin, J.M. et al (1979). Biochemistry 18, 5294).    mRNA is purified from this by oligo (dT) cellulose chromatography (Aviv, H. and Leder, P. (1972) PNAS 69, 1408).

First strand cDNA synthesis is done by oligo (dT)-primed reverse transcription (Retzel et al (1980) Biochemistry 19, 513-518) generating DNA/RNA hybrids. Second strands are synthesised by the action of RNase H₂¡ E.coli Polymerase I (large fragment) and E.coli ligase as described (Gubler, V. and Hoffman, B. (1983) Gene 25, 263-269) and the double stranded cDNA is tailed with poly (dC) ( Villa-Komaroff et al(1978) PNAS 75, 3727).    Tailed fragments are annealed into pBR322, cleaved and poly (dG) tailed at the Pst I site.    These hybrids are transformed into E.coli DH1 competent for transformation, and selected on agar plates for tetracycline resistance.    The transformants are screened by colony hybridisation on nitro-celluloseside filters    (Hanahan, D. and Meselson, M. (1980) Gene 10, 63-67).    The hybridisation probe is a DNA fragment containing the coding region for rat lingual lipase labelled by nick translation (Rigby, P.W.J. et al (1977) J. Mol. Biol. 113, 237).    The human lingual lipase is sufficiently homologous to rat lingual lipase to allow detection by DNA hybridisation.

Putative human lingual lipase clones are mapped by restriction endonucleases and subjected to DNA sequencing as described for rat lingual lipase.    A full length cDNA clone of human pre lingual lipase or met lingual lipase is expressed in E.coli, yeast or animal cells as described above for rat lingual lipase.    Expressed human lingual lipase is detected, assayed, characterised and purified from E.coli, yeast or animal cells in tissue culture as described above for rat lingual lipase.

It will of course be understood that the present invention has been described above purely by way of example, and modifications of detail can be made within the scope and spirit of the invention.

CLAIMS:

1. A lingual lipase protein for use in the treatment of lipase deficiency.

2. A lingual lipase protein according to claim 1 wherein the lingual lipase protein is a human lingual lipase protein.

3. A lingual lipase protein according to claim 1 wherein the lingual lipase protein is a rat lingual lipase protein.

4. A process for the production of a methionine-lingual lipase protein comprising producing the protein in a host organism transformed with a vector including a gene coding for the methionine-lingual lipase protein.

5. A process for the production of a lingual lipase protein comprising producing a lingual lipase precursor protein in a host organism transformed with a vector including a gene coding for the precursor protein and cleaving the precursor protein, to produce the lingual lipase protein.

6. A process according to claim 5 wherein the lingual lipase precursor protein is a prelingual lipase protein and the host organism is a host organism capable of cleaving the prelingual lipase protein to produce the lingual lipase protein.

7. A process according to claim 6 wherein the host organism is a mammalian cell in tissue culture.

8. A process according to claim 5 wherein the lingual lipase precursor protein comprises a heterologous protein and a lingual lipase protein.

9. A prelingual lipase protein.

10. A methionine-lingual lipase protein.

11. A fusion protein comprising a lingual lipase protein and a heterologous protein.

12. A gene coding for a protein comprising at least the amino acid sequence of a lingual lipase protein.

13. A gene according to claim 12 coding for a protein according to any one of claims 1,2,3,9,10 and 11.

14. A vector including a gene according to claim 12 or 13.

15. A host organism transformed with a vector according to claim 14.

16. An antibody having specificity for an antigenic determinant of a lingual lipase protein.

17. A pharmaceutical composition comprising a lingual lipase protein and a pharmaceutically acceptable excipient.

18. A pharmaceutical composition according to claim 17 wherein the lingual lipase protein is a lingual lipase protein according to claim 2 or 3 produced by the process of claim 4 or 5.

19. A pharmaceutical composition according to claim 17 or 18 in unit dosage form.

20. A pharmaceutical composition according to claim 17 or 18 in a liquid form.

FIG. 1

FIG. 2

FIG. 4

FIG. 3

0131418

```
          -18                    -10                              +1  L F G K L G(M)G  N  P  E  A  N(V)N  I  S  Q  M  I
                                                                -1  L F G K L G(M)G  N  P  E  A  N(V)N  I  S  Q  M  I
          M  W  L  L  L  I  T  S  V  I  S  T  F  G  G  A  H  G   L  F  G  K  L  G(P)G  N  P  E  A  N(M)N  I  S  Q  M  I
TACAAGATGTGGCTGTTATTAATAACAAGTGTGATATCAACATTCGGAGGTGCACATGGCCTATTTGGAAAACTGGGTCCTGGAAACCCTGAAGCAAATATGAATATTAGTCAGATGATA
I                                                                                                          100

        T  Y  W  G  Y  P  -  Q  E  -  E  V
        T  Y  W  G  Y  P  C  Q  E  Y  E  V  V  T  E  D  G  Y  I  L  G  V  Y  R  I  F  H  Q  K  N  N  S  E  N  I  G  K  R  F  V
ACTTACTGGGGATATCCATGTCAAGAATATGAAGTTGTTACTGAAGATGGCTACATTCTGGGGGTCTACAGAATTCCTCATGGGAAGAATAATTCTGAAAATATAGGCAAGAGACCTGTG
                                                                    200

        V  Y  L  Q  H  Q  L  I  A  S  A  T  N  W  I  A  N  L  P  N  N  S  L  A  F  H  L  A  D  A  G  Y  D  V  W  L  G  N  S  R
GTGTATTTGCAGCATGGTTTGATCGCATCAGCCACAAACTGGATTGCAAATCTACCAAACAACAGCCTGGCCTTTATGCTAGCAGACGCTGGCTATGATGTGTGGCTGGGGAACAGTCGA
                                300

        G  N  T  W  S  R  K  N  V  Y  Y  S  P  D  S  V  E  F  W  A  F  S  F  D  E  M  A  K  Y  D  L  F  A  T  I  N  F  I  V  Q
GGAAATACATGGTCCAGGAAAAATGTATACTACTCACCAGACTCAGTTGAATTCTGGGCTTTCAGCTTTGATGAAATGGCTAAATATGACCTTCCCGCCACAATAAACTTCATTGTACAG
                                400

        K  T  G  Q  E  K  I  H  Y  V  G  H  S  Q  G  T  T  I  G  F  I  A  F  S  T  N  P  T  L  A  K  K  I  K  T  F  Y  A  L  A
AAAACTGGACAAGAGAAGATACACTATGTTGGTCATTCTCAGGGCACCACTATTGGTTTCATTGCCTTTTCTACCAATCCTACACTGGCCAAAAAAATCAAGACGTTTTATGCATTAGCT
                 500                                                                                                    600

        P  V  A  T  V  K  Y  T  Q  S  P  L  K  K  I  S  F  I  F  T  F  L  F  K  L  M  F  G  K  K  M  F  L  P  H  T  Y  F  D  D
CCAGTTGCTACCGTGAAGTATACACAAAGTCCCTTGAAAAAGATTTCATTTATTCCTACATTTCTTTTCAAGCTTATGTTTGGCAAGAAAATGTTCCTGCCCCACACCTACTTTGATGAC
                                         200                                                              700

        F  L  G  T  E  V  C  S  R  E  V  L  D  L  L  C  S  N  T  L  F  I  F  C  G  F  D  K  K  N  L  N  V  S  R  F  D  V  Y  L
TTTCTTGGTACCGAAGTGTGCTCACGGGAGGTTCTAGATCTTCTCTGCAGCAACACTTTATTCATCTTCTGTGGATTTGACAAGAAAAACTTAAATGTGAGTCGTTTTGATGTGTATCTA
                                            250                                                          800

        G  H  N  P  A  G  T  S  V  Q  D  F  L  H  W  A  Q  L  V  R  S  G  K  F  G  A  F  N  W  G  S  P  S  Q  N  M  L  H  Y  N
GGGCATAATCCAGCAGGGACATCTGTTCAAGACTTTCTCCACTGGGCACAGCTTGTTAGATCTGGGAAATTTCAAGCCTTCAACTGGGGAAGCCCATCCCAGAACATGTTACACTACAAC
                                                         300
                                            900

        Q  K  T  F  F  E  Y  D  V  S  A  H  T  V  P  V  A  V  W  N  G  G  N  D  I  L  A  D  P  Q  D  V  A  H  L  L  F  K  L  S
CAGAAAACGCCTCCTGAATATGATGTGTCAGCCATGACTGTGCCAGTTGCAGTGTGGAACGGTGGCAATGACATCCTGGCTGATCCCCAAGATGTCGCCATGCTGCTTCCAAAACTCTCC
                                        1000

        N  L  L  F  H  K  E  I  L  A  Y  N  H  L  D  F  I  W  A  M  D  A  P  Q  E  V  Y  N  E  M  I  S  M  M  A  E  D  *
AACCTCCTGTTCCATAAGGAGATTCTTGCCTACAATCACCTGGACTTCATCTGGGCAATGGATGCCCCTCAAGAGGTTTACAATGAGATGATTTCCATGATGGCAGAAGACTAAAAGAAT
                 350                                                       370                   377
                 1100                                                                                    1200

CAATTATCCACATCCATTCCAAAATCTTTTCCCCTTTCATACAGTTTTTTCCAGTGTTTAAAAATGTAATACATATTACTGTAATTTTGACTTTAGAAATATATTGGCATCAGCAAAAAAA
                                                                                                1300

AAAAAAAAAAAAAAAA
             1340
```

FIG. 5

0131418

FIG. 6

European Patent
Office

**EUROPEAN SEARCH REPORT**

**0131418**

Application number

EP 84 30 4469

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | THE JOURNAL OF BIOLOGICAL CHEMISTRY, vol. 254, no. 23, 10th December 1979, pages 12121-12125; M. HAMOSH et al.: "Rat lingual lipase" * Whole document * | 1 | C 12 N 15/00 C 12 P 21/02 C 12 N 9/20 A 61 K 37/54 C 12 N 1/16 |
| D,P X | THE JOURNAL OF BIOLOGICAL CHEMISTRY, vol. 258, no. 23, 10th December 1983, pages 14563-14569; R.B. FIELD et al.: "Purification and characterization of rat lingual lipase" * Whole document * | 1 | |
| A | CHEMICAL ABSTRACTS, vol. 97, 1982, page 240, no. 209601u, Columbus, Ohio, USA; M.L. VASIL et al.: "Cloning of a phosphate-regulation hemolysin gene (phospholipase C) from Pseudomonas aeruginosa" & J. BACTERIOL. 1982, 152(1), 431-440 * Abstract * | 1 | TECHNICAL FIELDS SEARCHED (Int. Cl. ³) C 12 N |

The present search report has been drawn up for all claims

| Place of search THE HAGUE | Date of completion of the search 15-10-1984 | Examiner DELANGHE L.L.M. |
|---|---|---|